Europäisches Patentamt

European Patent Office

Office européen des brevets

(11) Veröffentlichungsnummer: **0 350 852**
**A1**

## (12) EUROPÄISCHE PATENTANMELDUNG

(21) Anmeldenummer: 89112640.1

(22) Anmeldetag: 11.07.89

(51) Int. Cl.⁴: **C07C 31/20**

(30) Priorität: 14.07.88 DE 3823787

(43) Veröffentlichungstag der Anmeldung:
17.01.90 Patentblatt 90/03

(84) Benannte Vertragsstaaten:
AT BE CH DE ES FR GB IT LI LU NL SE

(71) Anmelder: BOEHRINGER INGELHEIM KG

D-6507 Ingelheim am Rhein(DE)
(84) BE CH DE ES FR IT LI LU NL SE AT

Anmelder: BOEHRINGER INGELHEIM
INTERNATIONAL G.M.B.H.

D-6507 Ingelheim am Rhein(DE)
(84) GB

(72) Erfinder: Müller, Klaus-Robert, Dr. Dipl.-Chem.
im Wörtel 44-46
D-6800 Mannheim 24(DE)

(54) Verfahren zur Herstellung von enantiomerenreinen Propan-1,2-diolen.

(57) Die Erfindung betrifft ein Verfahren zur Herstellung von enantiomerenreinem S(+)-Propan-1,2-diol sowie R(-)-Propan-1,2-diol aus L(-)-Lactid beziehungsweise D(+)-Lactid.

EP 0 350 852 A1

## Verfahren zur Herstellung von enantiomerenreinen Propan-1,2-diolen

Die Erfindung betrifft ein Verfahren zur Herstellung von enantiomerenreinen S(+)-Propan-,2-diol sowie R(-)-Propan-1,2-diol aus L(-)-Lactid beziehungsweise D(+)-Lactid.

Enantiomerenreine Propandiole dienen als wertvolle Reagenzien bei stereospezifischen Synthesen. So findet beispielsweise S(+)-Propan-1,2-diol bei der Herstellung des Calciumblockers Verapamil sowie von Gallopamil Verwendung [L.J. Theodore und W.L. Nelson, J. Org. Chem. 52 (1987) 1309].

Propandiole geringerer optischer Reinheit wurden bislang durch Reduktion der entsprechenden Milchsäureester mit komplexen Hydriden [J. Gambos, E. Haslinger und U. Schmidt, Chem. Ber. 109 (1976) 2645] oder Borwasserstoff [C. Melchiorre, Chem. Ind. (London) 1976, 218] hergestellt. Herstellungsverfahren, welche von Propylenglykolketalen ausgehen, liefern die entsprechenden Propan-1,2-diole in nur geringer Reinheit [P. Newman, Optical Resolution Procedures for Chemical Compounds, Vol. 3: Alcohols, Phenols, Thiols, Aldehydes and Ketones, Optical Resolution Information Center, Manhattan College, Riverdale, New York 1984, S. 23].

Die Aufgabe der vorliegenden Erfindung besteht darin, ein Verfahren zur Verfügung zu stellen, welches S(+)-Propan-1,2-diol sowie R(-)-Propan-1,2-diol in hoher optischer Reinheit liefert.

Erfindungsgemäß wird die Aufgabe dadurch gelöst, daß man L(-)-Lactid beziehungsweise D(+)-Lactid mit einem Reduktionsmittel umsetzt, nach an sich bekannten Methoden die Reaktionsmischung aufarbeitet und die Reaktionsprodukte isoliert.

Vorzugsweise wird die Reaktion in inerten Lösungsmitteln durchgeführt. Die resultierende Reaktionsmischung wird anschließend hydrolytisch aufgearbeitet und die entstandenen Propan-1,2-diole werden - nach dem Abtrennen der festen Reaktionsprodukte - auf dem Wege der Destillation isoliert.

Im Gegensatz zu den aus dem Stand der Technik bekannten Verfahren zur Herstellung von enantiomerenreinen Propan-1,2-diolen erhält man bei der Anwendung des erfindungsgemäßen Verfahrens S(+)-Propan-1,2-diol beziehungsweise R(-)-Propan-1,2-diol in einer höheren optischen Reinheit, was den direkten Einsatz der erfindungsgemäß hergestellten Propan-1,2-diole in stereospezifischen Synthesen ermöglicht.

Als Ausgangsmaterial werden L(-)-Lactid beziehungsweise D(+)-Lactid in einer optischen Reinheit größer 99,5 % eingesetzt.

Als Reduktionsmittel finden Borwasserstoff - wie der Boran-Dimethylsulfid-Komplex - sowie komplexe Hydride -wie Lithiumtetrahydridoboranat (LiBH$_4$). Vitride$^R$ (NaAlH$_2$(OCH$_2$CH$_2$OCH$_3$)$_2$) Natriumtetrahydridoboranat (NaBH$_4$), Lithiumaluminiumhydrid (LiAlH$_4$) und vorzugsweise Diisobutylaluminiumhydrid (DIBAH) sowie Silane - wie z.B. Triethoxysilan (HSi(OCH$_2$CH$_3$)$_3$) - Verwendung.

Bei der Reduktion des L(-)-Lactids beziehungsweise des D(+)-Lactids wird das Reduktionsmittel vorteilhafterweise im Überschuß eingesetzt. Vorzugsweise werden bei der Verwendung von Diisobutylaluminiumhydrid 4,1 bis 4,3 Mol - oder die entsprechende Menge eines anderen Reduktionsmittels -pro Mol L(-)-Lactid bzw. D(+)-Lactid eingesetzt.

Als inerte Lösungsmittel dienen zweckmäßigerweise aromatische oder aliphatische Kohlenwasserstoffe, wie zum Beispiel Toluol, Xylol oder Heptan oder mit Wasser nicht mischbare Ether wie Di-n-butylether und Anisol.

Die Reduktion erfolgt bei der Verwendung von komplexen Hydriden in Abhängigkeit von der Reaktivität des eingesetzten Hydrids in einem Temperaturbereich von Raumtemperatur bis zum Siedepunkt des Reaktionsgemisches und vorzugsweise in einem Temperaturintervall von 40°C bis 70°C.

Das L(-)-Lactid beziehungsweise D(+)-Lactid wird zweckmäßigerweise unter Inertgasatmosphäre in einem inerten Lösungsmittel suspendiert und unter Rühren langsam mit dem Reduktionsmittel, welches in einem inerten Lösungsmittel gelöst oder suspendiert sein kann, versetzt und eine zur Vervollständigung der Reduktion ausreichende Zeit lang nachgerührt. Die Zugabe der Reaktanden kann auch in inverser Reihenfolge erfolgen. Die Temperatur kann mit einer Thermostatisierungsvorrichtung in dem zur Reduktion erforderlichen Bereich gehalten werden.

Anschließend wird die Reaktionsmischung mit einer basisch reagierenden wässerigen Lösung einer geeigneten Alkali- oder Erdalkaliverbindung - beispielsweise Natronlauge - hydrolysiert und bei erhöhter Temperatur zur Vervollständigung der Hydrolyse nachgerührt.

Darauf wird die organische Phase abgetrennt, und - bei Verwendung von Alkali-Aluminiumhydriden - der pH-Wert der wässerigen Phase mit einer Mineralsäure oder einer organischen Säure bzw. deren wässeriger Lösung zur Fällung und Abtrennung des Aluminiumhydroxids auf einen Wert von ca. 6 eingestellt. Das Aluminiumhydroxid wird mit einer geeigneten Trennvorrichtung - beispielsweise mit einer mit Kieselgur oder Cellulose (Diacel$^R$) beschichteten Nutsche - abgetrennt und mit Wasser gewaschen.

Die Reaktionslösung bzw. die vereinigten Filtrate werden im Vakuum eingeengt. Der verbleibende Rückstand wird mit einem geeigneten Lösungsmittel, beispielsweise einem Alkohol, bevorzugt Ethanol oder Isopropanol, aufgeschlämmt, abgesaugt und gewaschen.

Das Lösungsmittel wird anschließend unter vermindertem Druck entfernt und der verbleibende Rückstand im Vakuum destilliert.

Die destillative Reinigung liefert S(+)-Propan-1,2-diol oder R(-)-Propan-1,2-diol als farblose Öle.

Die nachfolgenden Beispiele dienen zur Erläuterung der Erfindung ohne sie einzuschränken.

Beispiel 1:

S(+)-Propan-1,2-diol

43,2 g L(-)-Lactid (0,3 Mol) [optische Reinheit größer 99,5 %] werden in 200 ml Toluol suspendiert und unter Rühren langsam mit 1038 ml einer 20 proz. Lösung (1,24 Mol) von Diisobutylaluminiumhydrid in Toluol versetzt. Die Temperatur der Reaktionsmischung wird mittels einer Kühlmischung aus Trockeneis und Isopropanol in einem Intervall von 40°C bis 50°C gehalten. Nach beendeter Zugabe läßt man das Reaktionsgemisch zur Vervollständigung der Reduktion noch 1 Stunde bei 40°C bis 50°C nachrühren. Anschließend hydrolysiert man das Gemisch mit 840 ml 3 N Natronlauge, wobei die Reaktionsmischung über einen Zeitraum von 40 Minuten der wässerigen Lösung der Natronlauge zugefügt wird und die Temperatur während der Zugabe im Bereich von 75°C bis 80°C gehalten wird.

Zum Vervollständigen der Hydrolyse läßt man 30 Minuten in einem Temperaturbereich von 65°C bis 80°C nachrühren und stellt anschließend mit 300 g einer 32 Gew.-proz. Lösung von Chlorwasserstoff in Wasser einen pH-Wert von 6 ein.

Das dabei ausfallende Aluminiumhydroxid wird über eine mit Kieselgur (Diacel^R) belegte Nutsche abgesaugt und mit 750 ml Wasser gewaschen. Das Filtrat wird unter vermindertem Druck eingeengt und der resultierende Rückstand mit 100 ml kaltem Ethanol aufgeschlämmt, abgesaugt und mit 50 ml Ethanol gewaschen.

Nach dem Abdestillieren des Lösungsmittels im Vakuum erhält man ein gelb-braun gefärbtes Öl, welches nach der Destillation unter einem vermindertem Druck von 1 bis 2 hPa in einem Siedebereich von 50°C bis 60°C 31,3 g eines farblosen Öles liefert, welches einen spezifischen Drehwert von $[\alpha]_D20 = 17,3°$ aufweist in einer Ausbeute von 69 % d. Th..

Beispiel 2

Die Herstellung von R(-)-Propan-1,2-diol erfolgt analog Beispiel 1, wobei anstelle des L(-)-Lactids D(+)-Lactid als Ausgangsmaterial eingesetzt wird.

Man isoliert 31,3 g (69 % d. Th.) des R(-)-Propandiols als farbloses Öl mit einem Drehwert Von $[\alpha]_D20 = -17,2°$.

## Ansprüche

1) Verfahren zur Herstellung von enantiomerenreinem S(+)-Propan-1,2-diol und R(-)-Propan-1,2-diol, dadurch gekennzeichnet, daß man L(-)-Lactid bzw. D(+)-Lactid mit einem Reduktionsmittel umsetzt, und das Reaktionsprodukt isoliert.

2) Verfahren zur Herstellung von enantiomerenreinem S(+)-Propan-1,2-diol und R(-)-Propan-1,2-diol, dadurch gekennzeichnet, daß man L(-)-Lactid bzw. D(+)-Lactid mit einem Reduktionsmittel in einem inerten Lösungsmittel bei einer Temperatur zwischen 40°C und 70°C umsetzt, anschließend das Reaktionsgemisch hydrolytisch aufarbeitet, das inerte Lösungsmittel von der wässerigen Lösung abtrennt, gegebenenfalls ausfallende Aluminiumhydroxide durch Fällung abtrennt, die wässerige Phase einengt, den resultierenden Rückstand mit einem geeigneten Lösungsmittel aufschlämmt, die festen Reaktionsprodukte abfiltriert und das Filtrat nach dem Entfernen des Lösungsmittels einer destillativen Reinigung unterzieht.

3) Verfahren nach Anspruch 1 oder 2, dadurch gekennzeichnet, daß als Reduktionsmittel Borane, Borane in komplex gebundener Form, Aluminiumhydride sowie komplexe Bor- oder Aluminiumhydride oder Silane eingesetzt werden.

## Europäisches Patentamt

# EUROPÄISCHER RECHERCHENBERICHT

Nummer der Anmeldung

EP 89112640.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| X | CHEMICAL ABSTRACTS, Band 85, Nr. 11, 13. September 1976, Columbus, Ohio, USA MELCHIORE, CARLO "A convenient synthesis of S(+)--propane-1,2-diol" Seite 500 * Zusammenfassung-Nr. 77 544t * | 1-3 | C 07 C 31/20 |
| D,X | & Chem. Ind. (London) 1976, (5), 218 -- | | |
| A | CHEMICAL ABSTRACTS, Band 78, Nr. 11, 19. März 1973, Columbus, Ohio, USA GUETTE, J.P. et al. "Preparation and chiroptical properties of alpha-glycols" Seite 373 * Zusammenfassung-Nr. 70 871u & Bull. Soc. Chim. Fr. 1972, (11), 4217-24 -- | 1 | |
| A | PATENT ABSTRACTS OF JAPAN, unexamined applications, C Field, Band 1, Nr. 166, 27. Dezember 1977 THE PATENT OFFICE JAPANESE GOVERNMENT Seite 3595 C 77 * Kokai-Nr. 52-105 110 (SAGAMI CHUO) * -- | 1 | |
| A | US - A - 3 737 463 (WILLIAM L. HOWARD et al.) * Anspruch 1 * -- | 1 | |
| A | PATENT ABSTRACTS OF JAPAN, unexamined applications, | 1 | |

RECHERCHIERTE SACHGEBIETE (Int. Cl.4)

C 07 C 31/00
C 07 C 29/00
C 25 B

Der vorliegende Recherchenbericht wurde für alle Patentansprüche erstellt.

| Recherchenort | Abschlußdatum der Recherche | Prüfer |
|---|---|---|
| WIEN | 10-10-1989 | REIF |

Europäisches
Patentamt

Nummer der Anmeldung

-2-
EP 89112640.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | C Field, Band 11, Nr. 13, 14. Jänner 1987 THE PATENT OFFICE JAPANESE GOVERNMENT Seite 161 C 397 * Kokai-Nr. 61-191 631 (PAICEL CHEM IND) * -- DE - A1 - 3 226 888 (BASF) * Anspruch; Beispiele 1,2 * ---- | 1 | |

RECHERCHIERTE
SACHGEBIETE (Int. Cl.4)

Der vorliegende Recherchenbericht wurde für alle Patentansprüche erstellt.

| Recherchenort | Abschlußdatum der Recherche | Prüfer |
|---|---|---|
| WIEN | 10-10-1989 | REIF |